# EUROPÄISCHE PATENTSCHRIFT

(11) **EP 2 215 526 B1**
(45) Veröffentlichungstag und Bekanntmachung des Hinweises auf die Patenterteilung: **28.09.2011**
(21) Anmeldenummer: 08855494.4
(22) Anmeldetag: 25.11.2008
(51) Int. Cl.: G03C 1/73, C07D 311/78, C09B 57/00, C09B 57/02

(54) **PHOTOCHROME BENZOPYRANE**
PHOTOCHROMIC BENZOPYRANES
BENZOPYRANES PHOTOCHROMES

(30) Priorität: 26.11.2007 DE 102007057108
(43) Veröffentlichungstag der Anmeldung: 11.08.2010
(73) Patentinhaber: Rodenstock GmbH, 80469 München (DE)
(72) Erfinder: MELZIG, Manfred, 82234 Wessling (DE); ROHLFING, Yven, 81547 München (DE); WEIGAND, Udo, 81247 München (DE)
(74) Vertreter: Müller-Boré & Partner Patentanwälte
(86) Internationale Anmeldenummer: PCT/EP2008/009991
(87) Internationale Veröffentlichungsnummer: WO 2009/068247

(56) Entgegenhaltungen:
- WO-A-2007/054240
- FR-A- 2 783 249
- FR-A- 2 815 034

## Beschreibung

Die vorliegende Erfindung betrifft photochrome Benzopyrane und deren Verwendung in Kunststoffen aller Art, insbesondere für ophthalmische Zwecke. Es handelt sich bei den erfindungsgemäßen Verbindungen um photochrome Benzopyran-Verbindungen, bei denen ein mehrkerniger Aromat mit der 5- und 6-Stellung eines Benzopyrans verknüpft ist, wobei die Verknüpfung in 6-Stellung direkt und die Verknüpfung in 5-Stellung über eine ein- oder zweiatomige Brücke erfolgt.

Seit langem sind verschiedene Farbstoffklassen bekannt, die bei Bestrahlung mit Licht bestimmter Wellenlängen, insbesondere Sonnenstrahlen, reversibel ihre Farbe wechseln. Dies geht darauf zurück, daß diese Farbstoffmoleküle durch Lichtenergie in einen angeregten Zustand übergehen, den sie bei Unterbrechung der Energiezufuhr wieder verlassen und in ihren Ausgangszustand zurückkehren. Zu diesen photochromen Farbstoffen gehören verschiedene Pyransysteme, die im Stand der Technik mit unterschiedlichen Grundsystemen und Substituenten bereits beschrieben wurden.

Pyrane, speziell Naphthopyrane und von diesen abgeleitete größere Ringsysteme, sind derzeit die am meisten bearbeitete Klasse photochromer Verbindungen. Obwohl bereits im Jahr 1966 erstmals zum Patent angemeldet (US 3,567,605), konnten erst in den 90er Jahren Verbindungen entwickelt werden, die für den Einsatz in Brillengläsern geeignet erschienen. Eine geeignete Verbindungsklasse von Pyranen sind zum Beispiel die 2,2-Diaryl-2H-naphtho[1,2-b]pyrane oder die 3,3-Diaryl-3H-naphtho[2,1-b]pyrane, die in angeregter Form verschiedene Färbungen, wie Gelb, Orange oder Rotorange, zeigen.

Als weitere Verbindungsklasse photochromer Verbindungen sind höher annellierte Pyrane von Interesse, die aufgrund ihres größeren Ringsystems längerwellig absorbieren und rote, violette und blaue Farbtöne ergeben. Diese können entweder von den 2H-Naphtho[1,2-b]pyranen oder den 3H-Naphtho[2,1-b]pyranen abgeleitete Systeme sein, die durch Annellierung an der f-Seite aus den jeweiligen Naphthopyran-Systemen hervorgehen.

Diarylchromene, insbesondere Naphthopyrane oder heterozyklisch annellierte Benzopyrane, die in 6-Stellung des Benzopyrans mit einem Phenylring oder allgemeiner einem aromatischen oder heteroaromatischen Ring substituiert sind, welcher zusätzlich über die 5-Stellung des Benzopyrans über mindestens ein Kohlenstoffatom, Sauerstoffatom oder Stickstoffatom verbrückt ist, sind derzeit die vielversprechendsten photochromen Verbindungen.

Wird diese Verbrückung nur über ein Atom erzeugt, so ergibt sich ein an das Benzopyran annellierter Fünfring. Beispiele finden sich für ein Kohlenstoffatom in US 5,645,767, US 5,723,072 sowie US 5,955,520 und für ein Sauerstoffatom in US 6,018,059.

In US 5,723,072 kann zusätzlich an diesem Grundsystem ein un-, mono- oder disubstituierter heterozyklischer Ring an der g-, h-, i-, n-, o- oder p-Seite des Indenonaphthopyrans annelliert sein. Es werden demzufolge Indeno[1,2-f]naphtho[1,2-b]pyrane mit einer sehr großen Variationsbreite an möglichen Substituenten offenbart.

In WO 96/14596, WO 99/15518, US 5,645,767, WO 98/32037 und US 5,698,141 werden vom 2H-Naphtho[1,2-b]pyran abgeleitete photochrome indenoannellierte Naphthopyran-Farbstoffe, die sie enthaltenden Zusammensetzungen sowie ein Verfahren zu ihrer Herstellung offenbart. In US 5,698,141 kann zusätzlich an diesem Grundsystem ein un-, mono- oder disubstituierter heterozyklischer Ring an der g-, h-, i-, n-, o- oder p-Seite des Indenonaphthopyrans annelliert sein. Von der jeweils sehr umfangreichen Substituentenliste sind auch ganz spezielle Spiro-Verbindungen umfaßt, und zwar solche Systeme mit einer spiroheterozyklischen Gruppe, worin einschließlich des Spiroatoms an der 13-Position des Grundsystems ein 5- bis 8-gliedriger Ring, der stets zwei Sauerstoffatome enthält, vorhanden ist. Eine weitere Ausführungsform des Spiroringes findet sich in der japanischen Anmeldung 344762/2000.

Wird diese Verbindung über zwei Atome erzeugt, so ergibt sich ein annellierter Sechsring mit allein für C, O und N vielfältigen Möglichkeiten. Verbindungen mit C=O und N-R (Lactam-Brücke) werden in US 6,379,591 beschrieben. Verbindungen mit einer unsubstituierten CH₂-CH₂-Brücke sowie einem annellierten Heterocyclus in 7,8-Stellung des zugrundeliegenden Benzopyrans sind in US 6,426,023 offenbart.

US 6,506,538 beschreibt die carbocyclischen Analogverbindungen, bei denen die H-Atome in der Brücke durch OH, (C₁-C₆) Alkoxy oder zwei H-Atome an einem C-Atom durch =O ersetzt sein können. Alternativ kann auch eines der Kohlenstoffatome in der zweigliedrigen Brücke durch Sauerstoff ersetzt sein. Diese Verbindungen sind neben weiteren in WO 00/02884 beschrieben.

Wird diese Verbindung durch drei Atome erzeugt, ergibt sich ein annellierter 7-Ring mit sehr vielen Variationsmöglichkeiten durch Einfügen von Heteroatomen. Verbindungen mit einer CH₂-CH₂-CH₂-Brücke sind in US 6,558,583 beschrieben. Auch hier können die H-Atome in der Brücke durch OH, (C₁-C₆)-Alkyl oder (C₁ - C₆)-Alkoxy oder zwei Wasserstoffatome an einem C-Atom durch =O ersetzt sein. Sie absorbieren bei gleichem Substitutionsmuster kürzerwellig als die annellierten 6-Ringe.

US 2004/0094753 beschreibt sowohl Verbindungen mit 2- wie mit 3-atomiger Brücke. Die zweiatomige (Kohlenstoff-)Brücke ist dabei zusätzlich mit einem Carbo- bzw. Heterocyclus annelliert. Die dreiatomige Brücke enthält drei C-Atome oder zwei C-Atome und ein O-Atom ohne zusätzliche Annellierung. Beide Ringe können vielfältige Substituenten tragen.

Die verschiedenen, im Stand der Technik verfügbaren photochromen Farbstoffe haben jedoch Nachteile, die bei der Verwendung in Sonnenschutzgläsern den Tragekomfort des Brillenträgers wesentlich beeinträchtigen. Zum einen weisen die Farbstoffe eine nicht ausreichend langwellige Absorption im angeregten wie im nicht angeregten Zustand auf. Zum anderen liegt häufig eine zu hohe Temperaturempfindlichkeit der Eindunkelung vor, wobei gleichzeitig eine zu langsame Aufhellung eintreten kann. Darüber hinaus besitzen die im Stand der Technik verfügbaren Farbstoffe oft eine ungenügende Lebensdauer und erlauben damit nur eine geringe Haltbarkeit der Sonnenschutzgläser. Letzteres macht sich in schnell nachlassender Leistung und/oder starker Vergilbung bemerkbar.

Der vorliegenden Erfindung liegt daher die Aufgabe zugrunde, weitere photochromer Verbindung bereitzustellen, die sich durch die Kombination von langwelligem Absorptionsmaximum der geschlossenen Form mit steiler Kante zum sichtbaren Wellenlängenbereich, hoher Eindunkelungsleistung, sehr schneller Aufhellreaktion und sehr guter Lichtbeständigkeit auszeichnen sollen.

Diese Aufgabe wird durch die in den Ansprüchen gekennzeichneten Gegenstände gelöst.

Insbesondere werden photochrome Benzopyrane mit der allgemeinen Formel (I) bereitgestellt: 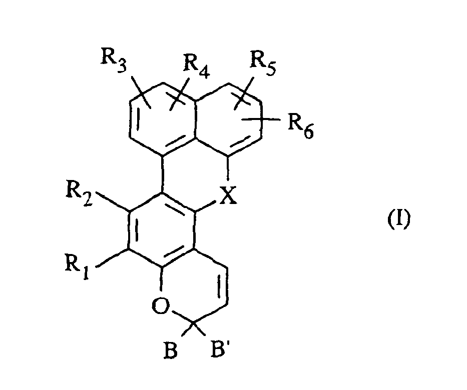 worin
die Reste R₁, R₂, R₃, R₄, R₅ und R₆ jeweils unabhängig voneinander einen Substituenten darstellen, ausgewählt aus der Gruppe α; bestehend aus einem Wasserstoffatom, einem (C₁-C₆)-Alkylrest, einem (C₁-C₆)-Thioalkylrest, einem (C₃-C₇)-Cycloalkylrest, der ein oder mehrere Heteroatome, wie beispielsweise O oder S, aufweisen kann, einem (C₁-C₆)-Alkoxyrest, einer Hydroxygruppe, einer Trifluormethylgruppe, Brom, Chlor, Fluor, einem un-, mono- oder disubstituiertem Phenyl-Phenoxy-, Benzyl-, Benzyloxy-, Naphthyl- oder Naphthoxyrest, wobei die Substituenten wiederum aus der Gruppe α ausgewählt sein können;
oder
die Reste R₁ und R₂ bzw. R₃ und R₄ (wenn zueinander in ortho-Stellung) bzw. R₄ und R₅ (sofern jeweils in peri-Stellung) bzw. R₅ und R₆ (wenn zueinander in ortho-Stellung) jeweils unabhängig voneinander eine an den aromatischen Ring gebundene -A-(CH₂)ₖ-D- Gruppe oder -A-(C(CH₃)₂)ₖ-D- Gruppe mit k = 1 oder 2 bilden, wobei A und D unabhängig voneinander aus Sauerstoff, Schwefel, CH₂, C(CH₃)₂ oder C(C₆H₅)₂ ausgewählt sind, und wobei an diese -A-(CH₂)ₖ-D- Gruppe wiederum ein Benzoring annelliert sein kann; oder
die Reste R₁ und R₂ bzw. R₃ und R₄ bzw. R₅ und R₆ jeweils unabhängig voneinander einen unsubstituierten, mono- oder disubstituierten annellierten Benzo-, Pyrido-, Naphtho-, Benzofuro- oder Benzothienoring darstellen, dessen Substituenten aus der Gruppe α ausgewählt sein können;
X eine ein- oder zweiatomige Brücke darstellt,
wobei X im Falle einer einatomigen Brücke aus O oder CR₇R₈ ausgewählt ist, wobei die Reste R₇ und R₈ jeweils unabhängig voneinander aus der Gruppe α ausgewählt sind, oder unter Einbeziehung des Spiro-Kohlenstoffatoms einen 3- bis 8-gliedrigen carbo- oder heteromonozyklischen Ring darstellen, der gegebenenfalls einen oder mehrere Substituenten aus der Gruppe α trägt und an den ein bis drei aromatische oder heteroaromatische Ringsysteme annelliert sein können, wobei das bzw. die Ringsysteme, unabhängig voneinander, aus der Gruppe β ausgewählt sind, bestehend aus Benzol, Naphthalin, Phenanthren, Pyridin, Chinolin, Furan, Thiophen, Pyrrol, Benzofuran, Benzothiophen, Indol und Carbazol, die wiederum mit einem oder mehreren Substituenten, ausgewählt aus der Gruppe α, substituiert sein können, oder unter Einbeziehung des Spiro-Kohlenstoffatoms, einen 7- bis 12-gliedrigen carbo-bizyklischen Ring bzw. einen 7- bis 12-gliedrigen carbo-trizyklischen Ring darstellen, der wiederum einen, zwei, drei oder vier Substituenten, ausgewählt aus der Gruppe α, aufweisen kann,
oder wobei X im Fall einer zweiatomigen Brücke aus der Gruppierung -Y-Z- gebildet wird, wobei Y und Z jeweils unabhängig voneinander aus O, CR₉R₁₀ und CR₁₁R₁₂ ausgewählt werden, wobei die Reste R₉ bis R₁₂ jeweils unabhängig voneinander aus der Gruppe α ausgewählt werden, oder R₉ und R₁₀ bzw. R₁₁ und R₁₂ unter Einbeziehung des Spiro-Kohlenstoffatoms einen 3- bis 8-gliedrigen carbo- oder heteromonozyklischen Ring darstellen, der gegebenenfalls einen oder mehrere Substituenten aus der Gruppe α trägt und an den ein bis drei aromatische oder heteroaromatische Ringsysteme annelliert sein können, wobei das bzw. die Ringsysteme, unabhängig voneinander, aus der Gruppe β ausgewählt sind, bestehend aus Benzol, Naphthalin, Phenanthren, Pyridin, Chinolin, Furan, Thiophen, Pyrrol, Benzofuran, Benzothiophen, Indol und Carbazol, die wiederum mit einem oder mehreren Substituenten, ausgewählt aus der Gruppe α, substituiert sein können, oder R₉ und R₁₀ bzw. R₁₁ und R₁₂ unter Einbeziehung des Spiro-Kohlenstoffatoms einen 7- bis 12-gliedrigen, carbo-bizyklischen Ring bzw. einen 7- bis 12-gliedrigen carbo-trizyklischen Ring darstellen, der wiederum einen, zwei, drei oder vier Substituenten, ausgewählt aus der Gruppe α, aufweisen kann;
oder Y und Z über jeweils einen Rest unter Ausbildung eines 4- bis 8-gliedrigen carbo-zyklischen Rings miteinander verbunden sind, wobei die Ringglieder, unabhängig voneinander, einen oder mehrere Substituenten, ausgewählt aus der Gruppe α, tragen können;
mit der Maßgabe, daß im Falle einer zweiatomigen Brücke nur maximal ein carbo-bi- oder carbo-trizyklisches System vorhanden ist und daß Y und Z nicht beide O darstellen; und

B und B' unabhängig voneinander aus einer der folgenden Gruppen a), b) oder c) ausgewählt sind, wobei
a) mono-, di- und trisubstituierte Arylreste sind, wobei der Arylrest Phenyl, Naphthyl oder Phenanthryl ist;
b) unsubstituierte, mono- und disubstituierte Heteroarylreste sind, wobei der Heteroarylrest Pyridyl, Furanyl, Benzofuranyl, Thienyl, Benzothienyl, 1,2,3,4-Tetrahydrocarbazolyl oder Julolidinyl ist;
   wobei die Substituenten der Aryl- oder Heteroarylreste in a) und b) solche sind, ausgewählt aus der vorstehend definierten Gruppe α oder der Gruppe χ, bestehend aus Hydroxy, am Phenylring un-, mono- oder disubstituiertem 2-Phenylethenyl, am Phenylring un-, mono- oder disubstituiertem (Phenylimino)methylen, am Phenylring un-, mono- oder disubstituiertem (Phenylmethylen)imino, Amino, Mono-(C₁-C₆)-alkylamino, Di-(C₁-C₆)-alkylamino, am Phenylring un-, mono- oder disubstituiertem Mono- und Diphenylamino, Piperidinyl, N-substituiertem Piperazinyl, Pyrrolidinyl, Imidazolidinyl, Pyrazolidinyl, Indolinyl, Morpholinyl, 2,6-Dimethylmorpholinyl, Thiomorpholinyl, Azacycloheptyl, Azacyclooctyl, un-, mono- oder disubstituiertem Phenothiazinyl, un-, mono- oder disubstituiertem Phenoxazinyl, un-, mono- oder disubstituiertem 1,2,3,4-Tetrahydrochinolinyl, un-, mono- oder disubstituiertem 2,3-Dihydro-1,4-benzoxazinyl, un-, mono- oder disubstituiertem 1,2,3,4-Tetrahydroisochinolinyl, un-, mono- oder disubstituiertem Phenazinyl, un-, mono- oder disubstituiertem Carbazolyl, un-, mono- oder disubstituiertem 1,2,3,4-Tetrahydrocarbazolyl und un-, mono- oder disubstituiertem 10,11-Dihydrodibenz[b,f]azepinyl, wobei der oder die Substituenten unabhängig voneinander wiederum aus (C₁-C₆)-Alkyl, (C₁-C₆)-Alkoxy, Brom, Chlor oder Fluor ausgewählt sein können;
   oder wobei zwei direkt benachbarte Substituenten eine U-(CV₂)ₚ-W-Gruppierung darstellen, wobei p = 1, 2 oder 3 ist, V Wasserstoff, CH₃ oder C₆H₅ sein kann und U und W unabhängig voneinander Sauerstoff, Schwefel, N-(C₁-C₆)-Alkyl, N-C₆H₅, CH₂, C(CH₃)₂ oder C(C₆H₅)₂ sein können, wobei zwei oder mehrere benachbarte Kohlenstoffatome dieser U-(CV₂)ₚ-W-Gruppierung jeweils unabhängig voneinander auch Teil eines daran annellierten Benzo-Ringsystems sein können, welches jeweils wiederum einen oder mehrere Substituenten, ausgewählt aus der Gruppe α oder der Gruppe χ, aufweisen kann;
   oder
c) B und B' zusammen mit dem benachbarten Kohlenstoffatom des Pyran-Ringes einen un-, mono- oder disubstituierten 9,10-Dihydroanthracen-, Fluoren-, Thioxanthen-, Xanthen-, Benzo[b]fluoren-, 5H-Dibenzo[a,d]cyclohepten oder Dibenzosuberon-Rest oder einen gesättigten Kohlenwasserstoffrest, der (C₃-C₁₂)-spiro-monozyklisch, (C₇-C₁₂)-spiro-bizyklisch bzw. (C₇-C₁₂)-spiro-trizyklisch ist, bilden, wobei die Substituenten der ungesättigten Cyclen unabhängig voneinander aus der Gruppe α oder der Gruppe χ ausgewählt sein können.

Die erfindungsgemäßen, von Benzopyranen abgeleiteten photochromen Verbindungen, bei denen ein mehrkerniger Aromat mit der 5- und 6-Stellung eines Benzopyrans verknüpft ist, wobei die Verknüpfung in 6-Stellung direkt und die Verknüpfung in 5-Stellung über eine ein- oder zweiatomige Brücke erfolgt, zeichnen sich durch sehr gute Lebensdauer sowie schnelle Aufhellungsgeschwindigkeit auf. Die erfindungsgemäßen Verbindungen zeigen gegenüber solchen ohne eine derartige Anbindung eines mehrkernigen Aromaten an die 5- und 6-Stellung eine deutlich längerwelliges Absorptionsmaximum. Die erfindungsgemäßen Verbindungen zeigen dabei eine ausgewogene Balance von langwelligem Absorptionsmaximum, hoher Eindunkelungsleistung, sehr schneller Aufhellreaktion und sehr guter Lichtbeständigkeit. Besonders hervorzuheben ist die erhöhte molare Absorption im kurzwelligen Bereich des sichtbaren Lichts um 400 nm.

Bevorzugte photochrome Benzopyrane gemäß der vorliegenden Erfindung weisen die nachfolgenden allgemeinen Formeln (II) bzw. (III) auf: 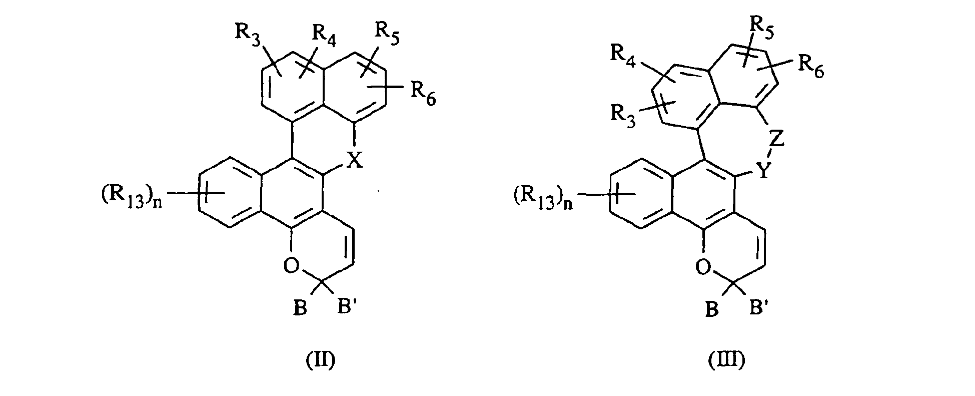 worin X für O oder CR₇R₈ steht, Y und Z wie vorgenannt definiert sind, und B, B', R₃, R₄, R₅, R₆, R₇ und R₈ wie vorgenannt definiert sind, R₁₃ aus der Gruppe α ausgewählt ist, und n 0, 1, 2, 3 oder 4 ist.

Wenn B bzw. B' für einen gesättigten Kohlenwasserstoffrest steht, der C₃-C₁₂ spiro-monozyklisch, C₇-C₁₂ spiro-bizyklisch oder C₇-C₁₂ spiro-trizyklisch ist, so werden unter C₃-C₁₂ spiro-monozyklisch ein dem Fachmann geläufiger 3-gliedriger bis 12-gliedriger Ring verstanden. Auch C₇-C₁₂ spiro-bizyklische Systeme sind einem Fachmann wohlbekannt. Beispielhaft kann hier wiederum Norbornan, Norbornen, 2,5-Norbornadien, Norcaran und Pinan genannt werden. Ein beispielhaftes C₇-C₁₂ spiro-trizyklisches System ist Adamantan.

In einer weiteren bevorzugten Ausführungsform sind die Reste B und B' unabhängig voneinander aus der Gruppe a), wie vorstehend definiert, ausgewählt.

Die Substituenten der Gruppe χ, welche Stickstoffatome aufweisen bzw. Amingruppen tragen, sind über diese an den Phenyl, Naphthyl- bzw. Phenanthrylrest der Gruppe a) gebunden.

Wenn bezüglich der Substituenten der Gruppe χ, welche an den Phenyl, Naphthyl- bzw. Phenanthrylrest der Gruppe a) für die Reste B bzw. B' gebunden sein können, zwei oder mehrere benachbarte Kohlenstoffatome dieser U-(CV₂)ₚ-W-Gruppierung jeweils unabhängig voneinander Teil eines daran annellierten Benzo-Ringsystems sein können, so bedeutet dies, dass dann die beiden Methylenkohlenstoffatome (-CH₂-CH₂-) Teil eines annellierten Ringsystems werden. Wenn beispielsweise zwei oder drei Benzoringe annelliert sind, so können beispielsweise hier dann folgende Struktureinheiten vorliegen, wie nachstehend angeführt.

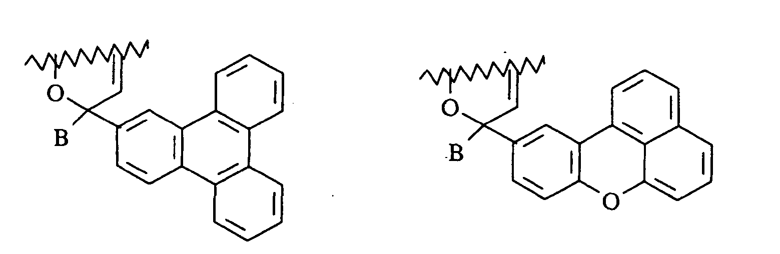

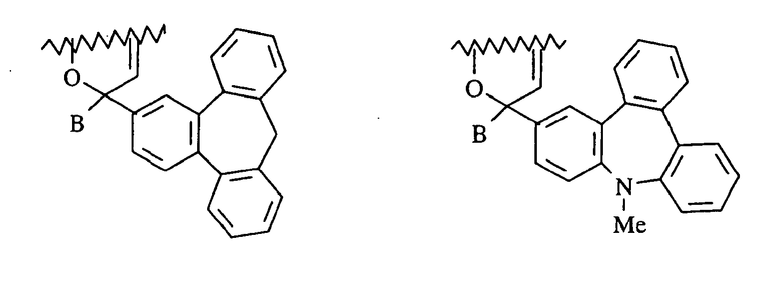

Selbstverständlich kann aber auch nur ein, über zwei benachbarte Kohlenstoffatome dieser U-(CV₂)ₚW-Gruppierung annellierter Benzoring vorliegen.

Wenn in den vorstehenden Formeln (I) bzw. (III) Y und Z über jeweils einen Rest unter Ausbildung eines 4- bis 8-gliedrigen carbo-zyklischen Rings miteinander verbunden sind, können beispielsweise Verbindungen gemäß Formel (IV), wie nachstehend aufgeführt, vorliegen, worin B, B', R₃, R₄, R₅, R₆, R₇ und R₈ wie vorgenannt definiert sind, R₁₃ und R₁₄ jeweils unabhängig voneinander aus der Gruppe α ausgewählt sind, und n jeweils unabhängig voneinander 0, 1, 2, 3 oder 4 ist:

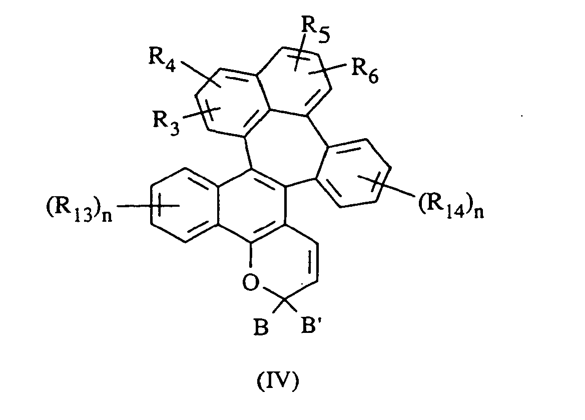

In einer bevorzugten Ausführungsform gemäß der vorliegenden Erfindung steht X in der Formel (I) für eine einatomige Brücke, ausgewählt aus O, CH₂ oder CMe₂.

In einer anderen bevorzugten Ausführungsform gemäß der vorliegenden Erfindung steht X in der Formel (I) für eine zweiatomige Brücke Y-Z, ausgewählt aus CH₂-CH₂, O-CH₂, CH₂-O oder einer Benzo-Annellierung.

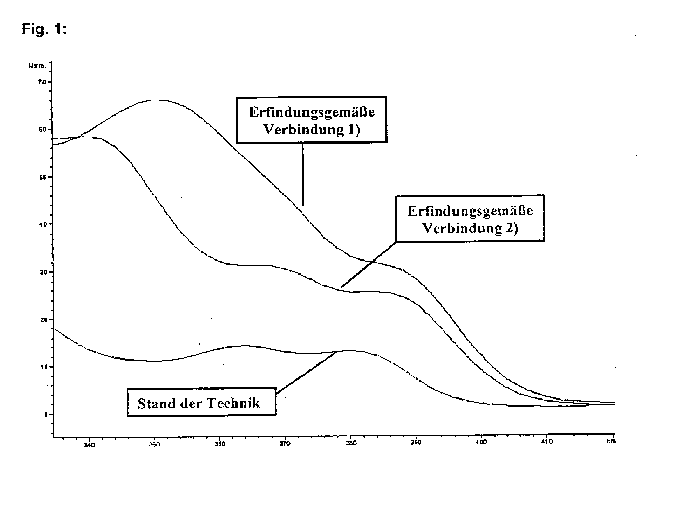

Im Vergleich zum Stand der Technik (US 6,506,538 bzw. US 6,558,583 für carbocyclische Brücken sowie WO 00/02884 für Brücken mit Sauerstoff), bei denen ein (substituierter) Benzol-Rest mit dem Benzopyran-Grundkörper verbrückt ist, weisen die erfindungsgemäßen Verbindungen - bei gleichen Substituenten B und B' - eine deutlich längerwellige Absorptionsbande sowohl im nicht angeregten als auch im angeregten Zustand auf. Eine längerwellige Absorption im nicht angeregten Zustand hat zwei wichtige Vorteile beim Einbringen der photochromen Farbstoffe z.B. in Kunststoff-Brillengläsern. Einerseits reagieren die erfindungsgemäßen Verbindungen auch dann, wenn bei ungünstigen atmosphärischen Bedingungen nur sehr langwelliges UV-Sonnenlicht (ab 380 nm) einfällt. Aus Figur 1 ist ersichtlich, daß die erfindungsgemäßen Verbindungen in der nicht angeregten Form bei Wellenlängen größer 370 nm im Vergleich zu Verbindungen des Standes der Technik deutlich intensiver absorbieren. Dadurch zeigen die erfindungsgemäßen photochromen Verbindungen auch bei ungünstigen Bedingungen eine sehr gute Eindunklungsleistung. Andererseits wird dadurch automatisch ein vollständiger UV-Schutz bis 400 nm erreicht, da die erfindungsgemäßen Verbindungen das einfallende UV-Licht komplett absorbieren - ein Zusatz von UV-Absorbern bei der Herstellung von Sonnenschutzgläsern erübrigt sich. Dies ist ein großer Vorteil, da zugemischte UV-Absorber immer auch einen Teil des Anregungslichts absorbieren, so daß Gläser mit UV-Absorber immer weniger stark eindunkeln als ohne UV-Absorber.

Die Struktur der in der Figur 1 dargestellten erfindungsgemäßen Verbindungen sowie deren längstwellige Absorptionsmaxima in der angeregten Form sind aus der folgenden Tabelle 1 ersichtlich (im Vergleich zum Stand der Technik aus US 6,558,583):
Tabelle 1: Längstwellige Absorptionsmaxima im angeregten Zustand

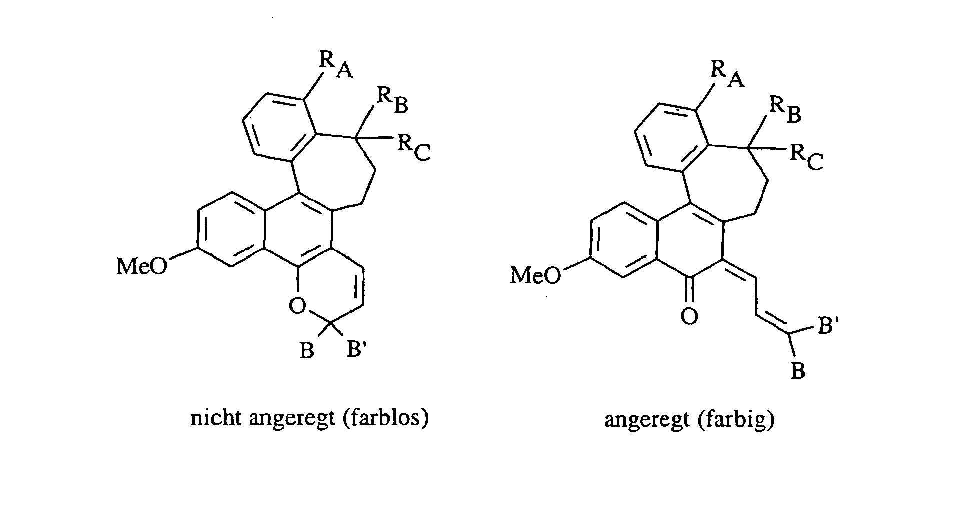

**A) Stand der Technik (US 6,558,583)**

| R_{A} | R_{B} | R_{C} | B | B' | λₘₐₓ (angeregt) |
|---|---|---|---|---|---|
| H | H | H | Phenyl | 4-(N-Morpholinyl)phenyl | 555 nm |

**B) Erfindungsgemäße Verbindungen**

| Verb. | R_{A} | R_{B} | R_{C} | B | B' | λₘₐₓ (angeregt) |
|---|---|---|---|---|---|---|
| 1) | Benzo-Annellierung * | | | Phenyl | 4-(N-Morpholinyl)phenyl | 580 nm |
| 2) | Benzo-Annellierung * | | | Phenyl | 4-(N-Morpholinyl)phenyl | 575 nm |

| | | | | | | |
|---|---|---|---|---|---|---|
| * die Benzo-Annellierung der erfindungsgemäßen Verbindung 1) weist noch einen Methoxy-Substituenten in ortho-Stellung zur Siebenring-Brücke auf, die der erfindungsgemäßen Verbindung 2) einen Methyl-Substituenten. | | | | | | |

Zur Messung der Eigenschaften der erfindungsgemäßen photochromen Farbstoffe sowie der Verbindung aus dem Stand der Technik (s.o.) wurden jeweils 500 ppm des Farbstoffes in einer Acrylatmonomer-Matrix gelöst und nach Zusatz eines Polymerisations-Initiators mit Hilfe eines Temperaturprogrammes thermisch polymerisiert. Die Transmisionseigenschaften der so hergestellten Kunststoffgläser (Dicke 2 mm) wurden anschließend nach DIN EN ISO 8980-3 vermessen.

Die erfindungsgemäßen Verbindungen können in Kunststoffmaterialien bzw. Kunststoffgegenständen jeglicher Art und Form für eine Vielzahl von Einsatzzwekken, für die photochromes Verhalten von Bedeutung ist, verwendet werden. Dabei können ein Farbstoff gemäß der vorliegenden Erfindung oder ein Gemisch solcher Farbstoffe eingesetzt werden. Beispielsweise können die erfindungsgemäßen photochromen Benzopyran-Farbstoffe in Linsen, insbesondere ophthalmischen Linsen, Gläsern für Brillen aller Art, wie beispielsweise Skibrillen, Sonnenbrillen, Motorradbrillen, Visieren von Schutzhelmen, und dergleichen eingesetzt werden. Ferner können die erfindungsgemäßen photochromen Benzopyrane beispielsweise auch als Sonnenschutz in Fahrzeugen und Wohnräumen in Form von Fenstern, Schutzblenden, Abdeckungen, Dächern oder dergleichen verwendet werden.

Zur Herstellung von solchen photochromen Gegenständen können die erfindungsgemäßen photochromen Benzopyrane durch verschiedene, im Stand der Technik beschriebene Verfahren, wie bereits in WO 99/15518 angegeben, auf ein Polymermaterial, wie ein organisches Kunststoffmaterial, aufgebracht oder darin eingebettet werden.

Es werden dabei sogenannte Massefärbungs- und Oberflächenfärbungsverfahren unterschieden. Ein Massefärbungsverfahren umfasst beispielsweise das Auflösen oder Dispergieren der photochromen Verbindung oder Verbindungen gemäß der vorliegenden Erfindung in einem Kunststoffmaterial, z.B. durch die Zugabe der photochromen Verbindung(en) zu einem monomeren Material, bevor die Polymerisation erfolgt. Eine weitere Möglichkeit zur Herstellung eines photochromen Gegenstands ist die Durchdringung des oder der Kunststoffmaterialien mit der (den) photochromen Verbindung(en) durch Eintauchen des Kunststoffmaterials in eine heiße Lösung des oder der photochromen Farbstoffe gemäß der vorliegenden Erfindung oder beispielsweise auch ein Thermotransferverfahren. Die photochrome(n) Verbindung(en) kann bzw. können beispielsweise auch in Form einer separaten Schicht zwischen aneinandergrenzenden Schichten des Kunststoffmaterials, z.B. als Teil eines polymeren Films, vorgesehen werden. Ferner ist auch ein Aufbringen der photochromen Verbindung(en) als Teil einer auf der Oberfläche des Kunststoffmaterials befindlichen Beschichtung möglich. Der Ausdruck "Durchdringung" soll dabei die Migration der photochromen Verbindung(en) in das Kunststoffmaterial, z.B. durch den lösungsmittelunterstützten Transfer der photochromen Verbindung(en) in eine Polymermatrix, Dampfphasentransfer oder andere derartige Oberflächendiffusionsvorgänge, bedeuten. Vorteilhafterweise können solche photochromen Gegenstände, wie z.B. Brillengläser, nicht nur mittels der üblichen Massefärbung, sondern in gleicher Weise auch mittels Oberflächenfärbung hergestellt werden, wobei bei der letzteren Variante eine überraschend geringere Migrationsneigung erzielt werden kann. Dies ist vor allem bei nachfolgenden Veredelungsschritten von Vorteil, da - z.B. bei einer Antireflexbeschichtung durch die geringere Rückdiffusion im Vakuum - Schichtablösungen und ähnliche Defekte drastisch verringert werden.

Insgesamt können auf Basis der erfindungsgemäßen photochromen Benzopyrane beliebig kompatible (in chemischer Hinsicht und farblicher Art und Weise verträgliche) Färbungen, d.h. Farbstoffe, auf das Kunststoffmaterial aufgebracht oder in es eingebettet werden, um sowohl ästhetischen Gesichtspunkten als auch medizinischen oder modischen Aspekten zu genügen. Der oder die spezifisch ausgewählte(n) Farbstoff(e) kann bzw. können demzufolge, abhängig von den beabsichtigten Wirkungen sowie Anforderungen, variieren.

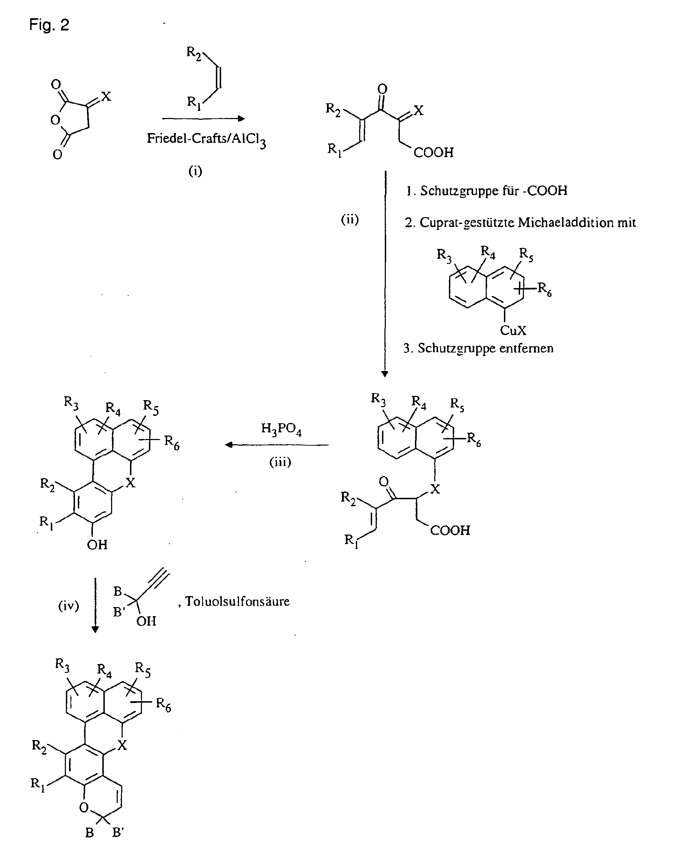

Die erfindungsgemäßen photochromen Verbindungen lassen sich gemäß folgendem beispielhaften Syntheseschema, wie in Figur 2 aufgezeigt, herstellen.

Entsprechende Succinanhydrid-Derivate werden in einem ersten Schritt einer Friedel-Crafts-Reaktion mit geeignet substituierten 1,2-Ethylenen unterworfen (Schritt (i)). Die -COOH Gruppe des daraus resultierenden Intermediats wird anschließend geschützt und dieses Intermediat einer Michael-Addition mit entsprechend substituierten Naphthalinderivaten unterworfen (Schritt (ii)). Nach Entfernung der Carbonsäure-Schutzgruppe werden via intramolekularer Cyclisierung mittels Phosphorsäure entsprechend substituierte Intermediate gebildet (Schritt (iii)). Anschließend werden diese substituierten Intermediate mit geeignet substituierten 2-Propin-1-ol-Derivaten gemäß Schritt (iv) zu den erfindungsgemäßen Verbindungen umgesetzt.

## Patentansprüche

1. Photochrome Benzopyrane mit der allgemeinen Formel (III): 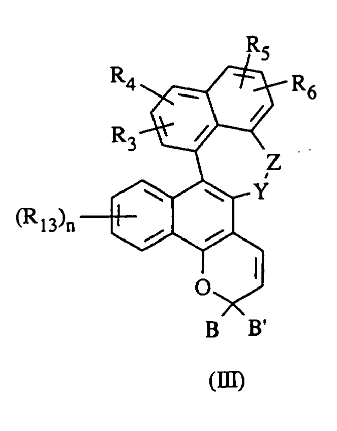 worin
die Reste R₃, R₄, R₅ und R₆ jeweils unabhängig voneinander einen Substituenten darstellen, ausgewählt aus der Gruppe α; bestehend aus einem Wasserstoffatom, einem (C₁-C₆)-Alkylrest, einem (C₁-C₆)-Thioalkylrest, einem (C₃-C₇)-Cycloalkylrest, der ein oder mehrere Heteroatome, wie beispielsweise O oder S, aufweisen kann, einem (C₁-C₆)-Alkoxyrest, einer Hydroxygruppe, einer Trifluormethylgruppe, Brom, Chlor, Fluor, einem un-, mono- oder disubstituiertem Phenyl-, Phenoxy-, Benzyl-, Benzyloxy-, Naphthyl- oder Naphthoxyrest, wobei die Substituenten wiederum aus der Gruppe α ausgewählt sein können;
oder
die Reste R₃ und R₄ bzw. R₄ und R₅ bzw. R₅ und R₆ jeweils unabhängig voneinander eine an den aromatischen Ring gebundene -A-(CH₂)ₖ-D- Gruppe oder -A-(C(CH₃)₂)ₖ-D- Gruppe mit k = 1 oder 2 bilden, wobei A und D unabhängig voneinander aus Sauerstoff, Schwefel, CH₂, C(CH₃)₂ oder C(C₆H₅)₂ ausgewählt sind, und wobei an diese -A-(CH₂)ₖ-D- Gruppe wiederum ein Benzoring annelliert sein kann; oder
die Reste R₃ und R₄ bzw. R₅ und R₆ jeweils unabhängig voneinander einen unsubstituierten, mono- oder disubstituierten annellierten Benzo-, Pyrido-, Naphtho-, Benzofuro- oder Benzothienoring darstellen, dessen Substituenten aus der Gruppe α ausgewählt sein können;
R₁₃ aus der Gruppe α ausgewählt ist, und n 0, 1, 2, 3 oder 4 ist;
wobei Y und Z jeweils unabhängig voneinander aus O, CR₉R₁₀ und CR₁₁R₁₂ ausgewählt werden, wobei die Reste R₉ bis R₁₂ jeweils unabhängig voneinander aus der Gruppe α ausgewählt werden, oder R₉ und R₁₀ bzw. R₁₁ und R₁₂ unter Einbeziehung des Spiro-Kohlenstoffatoms einen 3- bis 8-gliedrigen carbo- oder heteromonozyklischen Ring darstellen, der gegebenenfalls einen oder mehrere Substituenten aus der Gruppe α trägt und an den ein bis drei aromatische oder heteroaromatische Ringsysteme annelliert sein können, wobei das bzw. die Ringsysteme, unabhängig voneinander, aus der Gruppe β ausgewählt sind, bestehend aus Benzol, Naphthalin, Phenanthren, Pyridin, Chinolin, Furan, Thiophen, Pyrrol, Benzofuran, Benzothiophen, Indol und Carbazol, die wiederum mit einem oder mehreren Substituenten, ausgewählt aus der Gruppe α, substituiert sein können, oder R₉ und R₁₀ bzw. R₁₁ und R₁₂ unter Einbeziehung des Spiro-Kohlenstoffatoms einen 7- bis 12-gliedrigen, carbo-bizyklischen Ring bzw. einen 7- bis 12-gliedrigen carbo-trizyklischen Ring darstellen, der wiederum einen, zwei, drei oder vier Substituenten, ausgewählt aus der Gruppe α, aufweisen kann;
oder Y und Z über jeweils einen Rest unter Ausbildung eines 4- bis 8-gliedrigen carbo-zyklischen Rings miteinander verbunden sind, wobei die Ringglieder, unabhängig voneinander, einen oder mehrere Substituenten, ausgewählt aus der Gruppe α, tragen können;
mit der Maßgabe, daß nur maximal ein carbo-bi- oder carbo-trizyklisches System vorhanden ist und daß Y und Z nicht beide O darstellen; und
B und B' unabhängig voneinander aus einer der folgenden Gruppen a), b) oder c) ausgewählt sind, wobei
a) mono-, di- und trisubstituierte Arylreste sind, wobei der Arylrest Phenyl, Naphthyl oder Phenanthryl ist;
b) unsubstituierte, mono- und disubstituierte Heteroarylreste sind, wobei der Heteroarylrest Pyridyl, Furanyl, Benzofuranyl, Thienyl, Benzothienyl, 1,2,3,4-Tetrahydrocarbazolyl oder Julolidinyl ist;
wobei die Substituenten der Aryl- oder Heteroarylreste in a) und b) solche sind, ausgewählt aus der vorstehend definierten Gruppe α oder der Gruppe χ, bestehend aus Hydroxy, am Phenylring un-, mono- oder disubstituiertem 2-Phenylethenyl, am Phenylring un-, mono- oder disubstituiertem (Phenylimino)methylen, am Phenylring un-, mono- oder disubstituiertem (Phenylmethylen)imino, Amino, Mono-(C₁-C₆)-alkylamino, Di-(C₁-C₆)-alkylamino, am Phenylring un-, mono- oder disubstituiertem Mono- und Diphenylamino, Piperidinyl, N-substituiertem Piperazinyl, Pyrrolidinyl, Imidazolidinyl, Pyrazolidinyl, Indolinyl, Morpholinyl, 2,6-Dimethylmorpholinyl, Thiomorpholinyl, Azacycloheptyl, Azacyclooctyl, un-, mono- oder disubstituiertem Phenothiazinyl, un-, mono- oder disubstituiertem Phenoxazinyl, un-, mono- oder disubstituiertem 1,2,3,4-Tetrahydrochinolinyl, un-, mono- oder disubstituiertem 2,3-Dihydro-1,4-benzoxazinyl, un-, mono- oder disubstituiertem 1,2,3,4-Tetrahydroisochinolinyl, un-, mono- oder disubstituiertem Phenazinyl, un-, mono- oder disubstituiertem Carbazolyl, un-, mono- oder disubstituiertem 1,2,3,4-Tetrahydrocarbazolyl und un-, mono- oder disubstituiertem 10,11-Dihydrodibenz[b,f]azepinyl, wobei der oder die Substituenten unabhängig voneinander wiederum aus (C₁-C₆)-Alkyl, (C₁-C₆)-Alkoxy, Brom, Chlor oder Fluor ausgewählt sein können;
oder wobei zwei direkt benachbarte Substituenten eine U-(CV₂)ₚ-W-Gruppierung darstellen, wobei p = 1, 2 oder 3 ist, V Wasserstoff, CH₃ oder C₆H₅ sein kann und U und W unabhängig voneinander Sauerstoff, Schwefel, N-(C₁-C₆)-Alkyl, N-C₆H₅, CH₂, C(CH₃)₂ oder C(C₆H₅)₂ sein können, wobei zwei oder mehrere benachbarte Kohlenstoffatome dieser U-(CV₂)ₚ-W-Gruppierung jeweils unabhängig voneinander auch Teil eines daran annellierten Benzo-Ringsystems sein können, welches jeweils wiederum einen oder mehrere Substituenten, ausgewählt aus der Gruppe α oder der Gruppe χ, aufweisen kann; oder
c) B und B' zusammen mit dem benachbarten Kohlenstoffatom des Pyran-Ringes einen un-, mono- oder disubstituierten 9,10-Dihydroanthracen-, Fluoren-, Thioxanthen-, Xanthen-, Benzo[b]fluoren-, 5H-Dibenzo[a,d]cyclohepten oder Dibenzosuberon-Rest oder einen gesättigten Kohlenwasserstoffrest, der (C₃-C₁₂)-spiro-monozyklisch, (C₇-C₁₂)-spiro-bizyklisch bzw. (C₇-C₁₂)-spiro-trizyklisch ist, bilden, wobei die Substituenten der ungesättigten Cyclen unabhängig voneinander aus der Gruppe α oder der Gruppe χ ausgewählt sein können.

2. Photochrome Benzopyrane nach Anspruch 1, wobei die zweiatomige Brücke Y-Z aus CH₂-CH₂, O-CH₂, CH₂-O oder einer Benzo-Annellierung ausgewählt ist.

3. Photochrome Benzopyrane nach Anspruch 1 oder 2, wobei die Reste R₉ und R₁₀ bzw. R₁₁ und R₁₂ unter Einbeziehung des Spiro-Kohlenstoffatoms einen 5- bis 8-gliedrigen carbozyklischen Ring darstellen, der wiederum einen, zwei, drei oder vier Substituenten, ausgewählt aus der Gruppe α, aufweisen kann, wobei an den carbozyklischen Ring ein bis drei Benzo-Ringe annelliert sein können, die wiederum einen oder zwei Substituenten, ausgewählt aus der Gruppe α, aufweisen können.

4. Photochrome Benzopyrane nach Anspruch 1, wobei die für R₉ bis R₁₂ und B und B' genannten C₇-C₁₂-spiro-bizyklischen Systeme aus Norbornan, Norbornen, 2,5-Norbornadien, Norcaran und Pinan ausgewählt sind und das spiro-trizyklische System aus Adamantan ausgewählt ist, wobei die vorgenannten Spirosysteme jeweils wiederum einen, zwei, drei oder vier Substituenten, ausgewählt aus der Gruppe α, aufweisen können.

5. Photochrome Benzopyrane nach einem der Ansprüche 1 bis 4, wobei B und B' unabhängig voneinander aus der Gruppe a) ausgewählt sind.

6. Verwendung der photochromen Benzopyrane nach einem der Ansprüche 1 bis 5 in und auf Kunststoffmaterialien.

7. Verwendung nach Anspruch 6, wobei das Kunststoffmaterial eine ophthalmische Linse ist.

## Claims

1. Photochromic benzopyrans with the general formula (III): 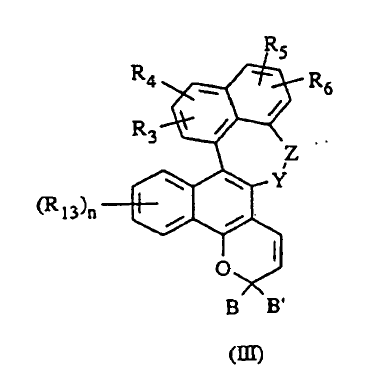 wherein
the radicals R₃, R₄, R₅ and R₆ represent respectively independently of each other substituents selected from the group α; consisting of a hydrogen atom, a (C₁-C₆)alkyl radical, a (C₁-C₆)thioalkyl radical, a (C₃-C₇)cycloalkyl radical, which can have one or more heteroatoms, such as for example O or S, a (C₁-C₆)alkoxy radical, a hydroxy group, a trifluoromethyl group, bromine, chlorine, fluorine, an un-, mono- or disubstituted phenyl-, phenoxy-, benzyl-, benzyloxy-, naphthyl- or naphthoxy radical, the substituents being able in turn to be selected from the group α;
or
the radicals R₃ and R₄ or R₄ and R₅ or R₅ and R₆ form respectively independently of each other an -A-(CH₂)ₖ-D- group or -A-(C(CH₃)₂)ₖ-D-group which is bonded to the aromatic ring with k = 1 or 2, A and D being selected independently of each other from oxygen, sulphur, CH₂, C(CH₃)₂ or C(C₆H₅)₂ and in turn a benzo ring being able to be anellated on this -A-(CH₂)ₖ-D- group; or
the radicals R₃ and R₄ or R₅ and R₆ represent respectively independently of each other an unsubstituted, mono- or disubstituted anellated benzo-, pyrido-, naphtho-, benzofuro- or benzothieno ring, the substituents of which ring can be selected from the group α;
R₁₃ is selected from the group α, and n is 0, 1, 2, 3 or 4;
Y and Z being selected respectively independently of each other from O, CR₉R₁₀ and CR₁₁R₁₂, the radicals R₉ to R₁₂ being selected respectively independently of each other from the group α, or R₉ and R₁₀ or R₁₁ and R₁₂ including the spiro carbon atom represent a 3- to 8-member carbo- or heteromonocyclic ring which carries possibly one or more substituents from the group α and on which ring one to three aromatic or heteroaromatic ring systems can be anellated, the ring system or systems being selected independently of each other from the group β, consisting of benzene, naphthalene, phenanthrene, pyridine, quinoline, furan, thiophene, pyrrole, benzofuran, benzothiophene, indole and carbazole, which in turn can be substituted with one or more substituents selected from the group α, or R₉ and R₁₀ or R₁₁ and R₁₂ including the spiro carbon atom represent a 7- to 12-member carbobicyclic ring or a 7- to 12-member carbotricyclic ring which in turn can have one, two, three or four substituents selected from the group α,
or Y and Z are connected to each other via respectively one radical with the formation of a 4 to 8-member carbocyclic ring, the ring members independently of each other being able to carry one or more substituents selected from the group α,
with the proviso that only a maximum of one carbo-bi- or carbotricyclic system is present and that Y and Z do not both represent O; and
B and B' are selected independently of each other from one of the following groups a), b) or c), wherein they
a) are mono-, di- and trisubstituted aryl radicals, the aryl radical being phenyl, naphthyl or phenanthryl;
b) are unsubstituted, mono- and disubstituted heteroaryl radicals, the heteroaryl radical being pyridyl, furanyl, benzofuranyl, thienyl, benzothienyl, 1,2,3,4-tetrahydrocarbazolyl or julolidinyl; the substituents of the aryl or heteroaryl radicals in a) and b) being those selected from the previously defined group α, or the group x consisting of hydroxy, on the phenyl ring un-, mono- or disubstituted 2-phenylethenyl, on the phenyl ring un-, mono- or disubstituted (phenylimino)methylene, on the phenyl ring un-, mono- or disubstituted (phenylmethylene)imino, amino, mono-(C₁-C₆)alkylamino, di-(C₁-C₆)alkylamino, on the phenyl ring un-, mono- or disubstituted mono- and diphenylamino, piperidinyl, N-substituted piperazinyl, pyrrolidinyl, imidazolidinyl, pyrazolidinyl, indolinyl, morpholinyl, 2-6-dimethylmorpholinyl, thiomorpholinyl, azacycloheptyl, azacyclooctyl, un-, mono- or disubstituted phenothiazinyl, un-, mono- or disubstituted phenoxazinyl, un-, mono- or disubstituted 1,2,3,4-tetrahydroquinolinyl, un-, mono- or disubstituted 2,3-dihydro-1,4-benzoxazinyl, un-, mono- or disubstituted 1,2,3,4-tetrahydroisoquinolinyl, un-, mono- or disubstituted phenazinyl, un-, mono- or disubstituted carbazolyl, un-, mono- or disubstituted 1,2,3,4-tetrahydrocarbazolyl and un-, mono- or disubstituted 10,11-dihydrodibenz[b,f]azepinyl, the substituent or substituents being able to be selected independently of each other in turn from (C₁-C₆)alkyl, (C₁-C₆)alkoxy, bromine, chlorine or fluorine;
or two directly adjacent substituents representing a U-(CV₂)ₚ-W-grouping, wherein p = 1, 2 or 3, V being able to be hydrogen, CH₃ or C₆H₅, and U and W being able to be independently of each other oxygen, sulphur, N-(C₁-C₆)alkyl, N-C₆H₅, CH₂, C(CH₃)₂ or C(C₆H₅)₂, two or more adjacent carbon atoms of this U-(CV₂)ₚ-W grouping being able to be respectively independently of each other also part of a benzo ring system anellated thereon, which respectively in turn can have one or more substituents selected from the group α or the group x;
or
c) B and B', together with the adjacent carbon atom of the pyran ring, form an un-, mono- or disubstituted 9,10-dihydroanthracene-, fluorene-, thioxanthene-, xanthene-, benzo[b]fluorene-, 5H-dibenzo[a,d]cycloheptene- or dibenzosuberone radical or a saturated hydrocarbon radical which is (C₃-C₁₂)spiromonocylic, (C₇-C₁₂)spirobicylic or (C₇-C₁₂)spirotricylic, the substituents of the unsaturated cycles being able to be selected independently of each other from the group α or the group x.

2. Photochromic benzopyrans according to claim 1, the two-atom bridge Y-Z being selected from CH₂-CH₂, O-CH₂, CH₂-O or a benzo-anellation.

3. Photochromic benzopyrans according to claim 1 or 2, the radicals R₉ and R₁₀ or R₁₁ and R₁₂ including the spiro carbon atom forming a 5- to 8-member carbocyclic ring, which in turn can have one, two, three or four substituents selected from the group α, one to three benzo rings being able to be anellated on the carbocyclic ring, which in turn can have one or two substituents selected from the group α.

4. Photochromic benzopyrans according to claim 1, the C₇-C₁₂ spirobicyclic systems mentioned for R₉ to R₈ and B and B' being selected from norbornane, norbornene, 2,5-norbornadiene, norcarane and pinane and the spirotricyclic system being selected from adamantane, the previously mentioned spirosystems respectively in turn being able to have one, two, three or four substituents, selected from the group α.

5. Photochromic benzopyrans according to one of the claims 1 to 4, B and B' being selected independently of each other from the group a).

6. Use of the photochromic benzopyrans according to one of the claims 1 to 5 in and on plastic materials.

7. Use according to claim 6, the plastic material being an ophthalmic lens.

## Revendications

1. Benzopyrane photochrome de formule générale (III) : 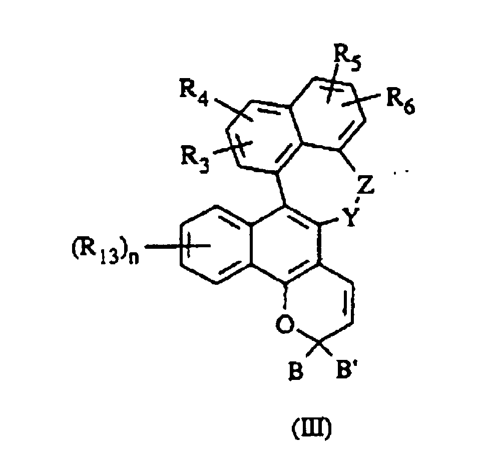 dans laquelle les radicaux R₃, R₄, R₅ et R₆ représentent respectivement, indépendamment les uns des autres, un substituant choisi dans le groupe α comprenant un atome d'hydrogène, un radical alkyle (en C₁ à C₆), un radical thioalkyle (en C₁ à C₆), un radical cycloalkyle (en C₃ à C₇) qui peut présenter un ou plusieurs hétéro-atomes tels que par exemple O ou S, un radical alcoxy (en C₁ à C₆), un groupe hydroxy, un groupe trifluorométhyle, un atome de brome, de chlore, de fluor, un radical phényle, phénoxy, benzyle, benzyloxy, naphtyle ou naphtoxy non substitué, mono ou disubstitué, les substituants pouvant à nouveau être choisis dans le groupe α ;
ou
les radicaux R₃ et R₄ ou R₄ et R₅ ou R₅ et R₆ forment respectivement indépendamment les uns des autres, un groupe -A-(CH₂)ₖ-D- ou un groupe -A-(C(CH₃)₂)ₖ-D- lié au cycle aromatique, où k = 1 ou 2, A et D étant choisis indépendamment l'un de l'autre parmi un atome d'oxygène, de soufre, CH₂, C(CH₃)₂ ou C(C₆H₅) et un cycle benzo pouvant être à nouveau annelé à ce groupe -A-(CH₂)ₖ-D- ; ou
les radicaux R₃ et R₄ ou R₅ et R₆ respectivement indépendamment les uns des autres, représentent un cycle benzo, pyrido, naphto, benzofuro ou benzothiéno non substitué, mono ou disubstitué, dont les substituants peuvent être choisis dans le groupe α ;
R₁₃ est choisi dans le groupe α et n est 0, 1, 2, 3 ou 4 ;
Y et Z étant choisis respectivement, indépendamment l'un de l'autre, parmi O, CR₉R₁₀ et CR₁₁R₁₂, les radicaux R₉ à R₁₂ étant choisis respectivement, indépendamment les uns des autres, dans le groupe α, ou R₉ et R₁₀ ou R₁₁ et R₁₂ en incluant l'atome de carbone spiro, représentent un cycle carbo- ou hétéromonocyclique de 3 à 8 chaînons qui porte éventuellement un ou plusieurs substituants dans le groupe α et auquel un à trois systèmes cycliques aromatiques ou hétéro-aromatiques peuvent être annelés, le ou les système(s) cyclique(s) étant choisis indépendamment les uns des autres dans le groupe β comprenant les groupes benzène, naphtaline, phénanthrène, pyridine, quinoline, furane, thiophène, pyrrol, benzofurane, benzothiophène, indole et carbazole qui peuvent à nouveau être substitués par un ou plusieurs substituants choisis dans le groupe α, ou R₉ et R₁₀ ou R₁₁ et R₁₂ en incluant l'atome de carbone spiro représentent un cycle carbo-bicyclique de 7 à 12 chaînons ou un cycle carbo-tricyclique de 7 à 12 chaînons qui peut présenter à nouveau un, deux, trois ou quatre substituants choisis dans le groupe α ;
ou Y et Z sont liés l'un à l'autre respectivement par un radical en formant un cycle carbo-cyclique de 4 à 8 chaînons, les chaînons du cycle pouvant porter indépendamment les uns des autres, un ou plusieurs substituants choisis dans le groupe α ;
à condition que seul un système carbo-bi- ou carbo-tricyclique soit présent et que Y et Z ne représentent pas tous deux 0 ; et
B et B' sont choisis indépendamment l'un de l'autre dans l'un des groupes suivants a), b) ou c), où
a) les radicaux aryle sont mono-, di- et trisubstitués, le radical aryle étant un groupe phényle, naphtyle ou phénantryle ;
b) les radicaux hétéroaryle sont non substitués, mono- et disubstitués, le radical hétéroaryle étant un groupe pyridyle, furanyle, benzofuranyle, thiényle, benzothiényle, 1,2,3,4-tétrahydrocarbazolyle ou julolidinyle ;
les substituants des radicaux aryle ou hétéroaryle dans a) et b) étant des substituants choisis dans le groupe α défini précédemment ou le groupe χ comprenant un groupe hydroxy, 2-phényléthényle non substitué, mono- ou disubstitué sur le cycle phényle, (phénylimino)méthylène non substitué, mono ou disubstitué sur le cycle phényle, (phénylméthylène)imino non substitué, mono ou disubstitué sur le cycle phényle, amino, mono-alkylamino (en C₁ à C₆), di-alkylamino (en C₁ à C₆), mono- et diphénylamino non substitué, mono ou disubsitué sur le cycle phényle, pipérazinyle N-substitué, pyrrolidinyle, imidazolidinyle, pyrazolidinyle, indolinyle, morpholinyle, 2,6-diméthylmorpholinyle, thiomorpholinyle, azacycloheptyle, azacyclo-octyle, phénothiazinyle non substitué, mono-ou disubstitué, phénoxazinyle non substitué, mono ou disubstitué, 1,2,3,4-tétrahydroquinolinyle non substitué, mono- ou disubstitué, 2,3-dihydro-1,4-benzoxazinyle non substitué, mono- ou disubstitué, 1,2,3,4-tétrahydro-isoquinolinyle non substitué, mono-ou disubstitué, phénazinyle non substitué, mono- ou disubstitué, carbazolyle non substitué, mono- ou disubstitué, 1,2,3,4-tétrahydrocarbazolyle non substitué, mono- ou disubstitué et 10,11-dihydrobenz[b,f]azépinyle non substitué, mono- ou disubstitué, le ou les substituant(s) pouvant être choisis indépendamment les uns des autres parmi les groupes alkyle (en C₁ à C₆), alcoxy (en C₁ à C₆), des atomes de brome, de chlore ou de fluor ;
où deux substituants directement voisins représentent un groupe U-(CV₂)ₚ-W- où p = 1, 2 ou 3, V pouvant être un atome d'hydrogène, CH₃ ou C₆H₅ et U et W peuvent être indépendamment l'un de l'autre, un atome d'oxygène, de soufre, un groupe N-alkyle (en C₁ à C₆), N-C₆H₅, CH₂, C(CH₃)₂ ou C(C₆H₅)₂, deux ou plusieurs atomes de carbone voisins de ce groupe U-(CV₂)ₚ-W-pouvant être respectivement indépendamment les uns des autres, également une partie d'un système benzocyclique annelé à celui-ci, qui peut présenter respectivement à nouveau un ou plusieurs substituants choisis dans le groupe α ou le groupe χ ; ou
c) B et B' peuvent former conjointement avec d'atome de carbone voisin du cycle pyrane, un radical 9,10-dihydroanthracène non substitué, mono- ou disubstitué, fluorène, thioxanthène, xanthène, benzo[b]fluorène, 5H-dibenzo[a,d]cycloheptène ou dibenzosubérone ou un radical hydrocarbure saturé qui est spiro-monocyclique (en C₃ à C₁₂), spiro-bicyclique (en C₇ à C₁₂) ou spiro-tricyclique (en C₇ à C₁₂), les substituants des cycles insaturés pouvant être choisis indépendamment les uns des autres dans le groupe α ou le groupe χ.

2. Benzopyrane photochrome selon la revendication 1, dans lequel le pont à deux atomes Y-Z est choisi parmi CH₂-CH₂, O-CH₂, CH₂-O ou un annelage benzo.

3. Benzopyrane photochrome selon la revendication 1 ou 2, dans lequel les radicaux R₉ et R₁₀ ou R₁₁ et R₁₂ en incluant l'atome de carbone spiro représentent un cycle carbocyclique de 5 à 8 chaînons qui peut présenter à nouveau un, deux, trois ou quatre substituants choisis dans le groupe α, un à trois cycles benzo pouvant être annelés au cycle carbocyclique qui peuvent présenter à nouveau un ou deux substituants choisis dans le groupe α.

4. Benzopyrane photochrome selon la revendication 1, dans lequel les systèmes spiro-bicycliques en C₇ à C₁₂ cités pour R₉ à R₁₂ et B et B' sont choisis parmi le norbormane, le norbornène, le 2,5-norbornadiène, le norcarane et le pinane et le système spiro-tricyclique est choisi dans l'adamantane, les systèmes spiro précédemment cités pouvant présenter respectivement à nouveau un, deux, trois ou quatre substituants choisis dans le groupe α.

5. Benzopyrane photochrome selon l'une des revendications 1 à 4, dans lequel B et B' sont choisis indépendamment dans le groupe a).

6. Utilisation du benzopyrane photochrome selon l'une des revendications 1 à 5 dans et sur des matières plastiques.

7. Utilisation selon la revendication 6, dans laquelle la matière plastique est une lentille ophtalmique.
